# EUROPÄISCHE PATENTSCHRIFT

(11) **EP 0 567 898 B1**
(45) Veröffentlichungstag und Bekanntmachung des Hinweises auf die Patenterteilung: **03.07.1996**
(21) Anmeldenummer: 93106376.2
(22) Anmeldetag: 20.04.1993
(51) Int. Cl.: A61B 5/11

(54) **Verfahren zur Messung der Körperhaltung und -bewegung**
Method for measurement of posture and body movements
Méthode de mesure de la posture et du mouvement du corps

(30) Priorität: 01.05.1992 DE 4214523
(43) Veröffentlichungstag der Anmeldung: 03.11.1993
(73) Patentinhaber: Manthey, Jürgen, Dr., D-07747 Jena (DE)
(72) Erfinder: Manthey, Jürgen, Dr., D-07747 Jena (DE)

(56) Entgegenhaltungen:
- WO-A-86/03392
- DE-A- 2 715 106
- FR-A- 2 449 433
- PROCEEDINGS OF THE ANNUAL INTERNATIONAL CONFERENCE OF THE IEEE ENGINEERING IN MEDICINE AND BIOLOGY SOCIETY Bd. 11, November 1989, US Seiten 1118 - 1119 JENSEN ET AL. 'A fiber optic angular sensor for biomedical applications'
- MEDICAL AND BIOLOGICAL ENGINEERING AND COMPUTING Bd. 15, Nr. 4, Juli 1977, STEVENAGE,GB Seiten 446 - 449 JACKSON 'Linearity of radio-frequency transducers.'

## Beschreibung

Die Erfindung betrifft ein Verfahren zur Messung der Körperhaltung und -bewegung.

Für verschiedene medizinische Anwendungen wie Diagnostik der Haltungsmuskulatur, Biofeedback der Körperhaltung zur Therapie von Haltungsfehlern, Erfassung der Gelenkstellung von Gliedmaßen sowie Ganganalysen ist die Messung von Körperhaltungen und -bewegungen erforderlich. Für diese Aufgabe finden Verfahren auf mechanischer (US 50 82 002, DD 251 075, EP 0154 102 A2), optischer, elektromagnetischer (DE-OS 1 541 180, DE 2 715 106) und Ultraschallbasis (WO 86/03 392 A1) Anwendung.

Die bekannten Meßsysteme zur Erfassung der Körperhaltung sind z.T. apparativ aufwendig (optische Systeme), erfordern im Raum angeordnete Sensoren (Ultraschall- sowie elektromagnetische Systeme), erlauben nur eine globale Bewertung mehrerer, kombiniert erfaßter Haltungsparameter (um den Körper gelegte Schlaufen zur Messung der Rumpflänge), beeinträchtigen den Patienten bei längerer Anwendungsdauer oder sind mechanisch relativ anfällig.

Es ist weiterhin ein elektromagnetisches System zur Erfassung der Winkelstellung von Körpergelenken bekannt, gemäß der Oberbegriffe der Ansprüche 1 und 7, bestehend aus einem Oszillator, der an den Gliedmaßen oberhalb des Gelenkes befestigt ist, und einer Sensorspule, fixiert unterhalb des Gelenkes (Med. & Biol. Eng. & Comput., 1977, 15, pg. 446-449). Die Stärke des in der Sensorspule induzierten Stromes ist ein Maß für den zweidimensional erfaßten Gelenkwinkel.

Der Erfindung liegt die Aufgabe zugrunde, ein universell einsetzbares Verfahren zur Messung von Körperhaltungen und -bewegungen zu schaffen, welches präzise arbeitet und bei der Anwendung am Patienten günstige Trageeigenschaften besitzt.

Diese Aufgabe wird erfindungsgemäß durch ein Verfahren nach Anspruch 1 und eine Vorrichtung nach Anspruch 7 gelöst.

Ein Beispiel für diese Bewegungsmessung ist die Erfassung des Kniewinkels über die Dehnung bzw. Kontraktion von Hautflächen. So wird die Haut über der Kniescheibe beim Anwinkeln des Beines gespannt, beim Strecken des Knies kontrahiert sie faltenfrei. Aber auch Lageänderungen der Körperteile selbst (z.B. Annäherung von Ober- und Unterschenkel beim Beugen des Kniegelenkes) rufen Abstandsänderungen fixer Hautpunkte hervor.

Eine Abstandsmessung auf der Haut kann gemäß Anspruch 2 mittels Ultraschallwandlern erfolgen. Aufgrund der Präzision der Ultraschalltechnik werden auch dann schon für die Messungen ausreichende, bewegungsinduzierte Abstandsvariationen der Ultraschallwandler beobachtet, wenn diese in relativ geringem Abstand (Größenordnung 1 cm) zueinander auf der Haut angeordnet sind.
Die Befestigung der Ultraschallwandler auf der Haut erfolgt durch Aufkleben, aber auch mittels elastischer Bänder.

Zur Erfassung der Ultraschallwandler-Abstände wird entsprechend Anspruch 3 vorzugsweise der Ultraschallweg über die oberflächlich gelegenen Körperschichten (Haut, Fettgewebe, Muskulatur) gewählt. Um eine Schallausbreitung auf dem kürzesten Weg zwischen den Ultraschallwandlern zu gewährleisten, ist die Schalleinkopplung unter einem flachen Winkel zur Hautoberfläche vorteilhaft.

Die Distanzmessung erfolgt auf einfache Weise mit kurzen Ultraschallimpulsen. Der zuerst am Ultraschallempfänger eintreffende Impuls wird für die Laufzeitmessung zugrunde gelegt. Dieses Prinzip funktioniert auch, wenn der Ultraschallweg zwischen Sender und Empfänger nicht exakt geradlinig ist, sondern Reflexionen beinhaltet.

Ein ausreichend breiter Schallfeldkegel wird erreicht, wenn der effektive Schallwandlerdurchmesser gegenüber der Ultraschallwellenlänge nicht zu groß gewählt wurde. Die Wahl der Ultraschallwellenlänge richtet sich nach dem für die Messung verwendeten Schallwandlerabstand. Große Wandlerabstände erfordern eine größere Wellenlänge, um die Ultraschallabsorption im Gewebe zu vermindern.

Ein bedeutendes Anwendungsgebiet des erfindungsgemäßen Verfahrens ist die Nutzung in einem Biofeedbackgerät zur Verbesserung der Körperhaltung und Prophylaxe von Wirbelsäulenbeschwerden. Bei dieser Anwendung wird der Abstandsmeßwert, der dem interessierenden Haltungsparameter (z.B. Schulterblätterabstand) entspricht, durch eine Elektronikeinheit beurteilt. Grundlage für diese Beurteilung ist ein vom Arzt festgelegter und elektronisch gespeicherter Normwert. Entspricht der Meßwert nicht den Anforderungen, wird eine Signalisierungseinrichtung, die dem Patienten die Notwendigkeit einer Haltungskorrektur anzeigt, angesteuert. Die Signalisierung kann auf akustischer, elektrischer, mechanischer, optischer oder thermischer Basis beruhen. Neben einem starr für das jeweilige Krankheitsbild festgelegten Normwert der Korperhaltung kann auch ein variables Kriterium, das den jeweiligen Therapiefortschritt sowie einen eventuellen Ermüdungszustand des Patienten berücksichtigt, zur Auslösung des Biofeedbacksignals dienen.

Neben der Anwendung einer Ultraschallmeßstrecke zur Abstandsermittlung vorbestimmter Hautpositionen kann die Distanz der auf der Haut des Patienten befestigten Sensoren gemäß Anspruch 4 auch mittels bekannter elektromagnetischer Verfahren erfolgen.

Eine Abstandsmessung mittels elektromagnetischer Verfahren ist möglich, indem man ein Sender-Empfänger-System verwendet, welches die abstandsabhängige Intensität einer elektromagnetischen Größe bestimmt. So kann die Stärke eines elektromagnetischen Hochfrequenzfeldes oder eines reinen Magnetfeldes (letzteres z.B. mittels Hallgeneratoren) gemessen werden.

Eine weitere Möglichkeit zur elektromagnetischen Abstandsermittlung ist die Messung der laufzeitabhängigen Phasendifferenz zwischen gesendetem und empfangenem Signal. Dabei ist eine in Relation zum gewählten Sender-Empfänger-Abstand angemessene Wellenlänge zu verwenden.

Für ein auf elektromagnetischer Basis beruhendes Meßsystem gelten die gleichen Anwendungsmöglichkeiten wie für ein Ultraschallsystem.

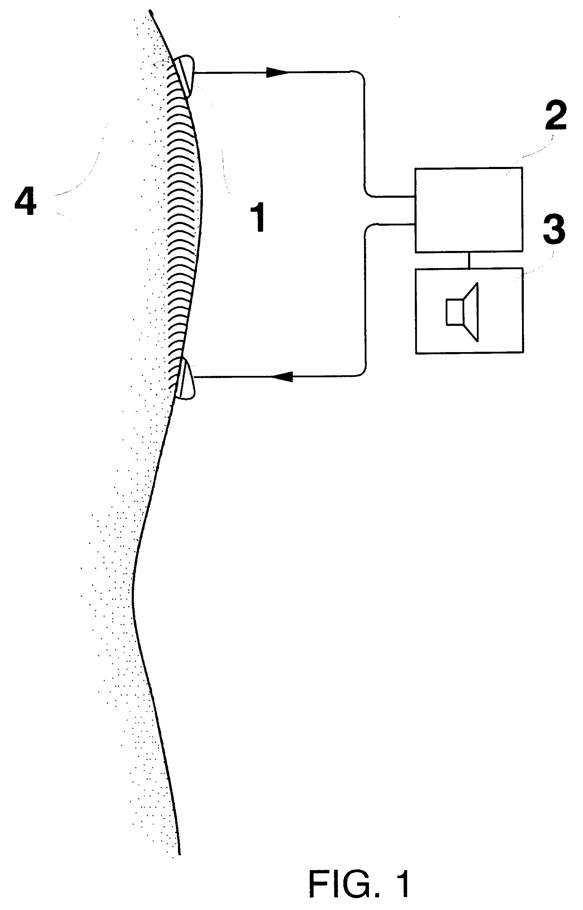

Im folgenden wird ein Beispiel zur Anwendung der Erfindung bei der Behandlung einer zu starken Krümmung der Brustwirbelsäule (Kyphose) mit Bezug auf die Ausführungsform des Verfahrens gemäß der beiliegenden Zeichnung näher beschrieben.

Die Ultraschallwandler **1** werden in vertikaler Anordnung auf vorbestimmte Hautpositionen **4** am Rücken des Patienten geklebt. Die Elektronikeinheit **2** mißt in kurzen zeitlichen Abständen die Laufzeit der Schallwellen von einem Ultraschallwandler zum anderen auf dem Weg durch den Körper. Ist die Ultraschall-Laufzeit durch eine zu starke Krümmung der Brustwirbelsäule größer als ein vom Arzt vorgegebener Wert, wird die Signalisierungseinrichtung **3** angesteuert. Diese löst ein akustisches Signal aus. Die Lautstärke des Tones ist anfangs gering (Erinnerungssignal) und steigert sich bis zur Wahrnehmung in der Umgebung des Patienten, wenn dieser seine Körperhaltung nicht rechtzeitig korrigiert.

Das am Korper des Patienten tragbare, mikroprozessorgesteuerte Gerät zur Anwendung des Verfahrens ist batteriebetrieben und auf Langzeitanwendung ausgelegt.

Prinzipiell ist auch der Ultraschallweg vom Ultraschallsender zum -empfänger über die Luft zur Messung geeignet. Allerdings wird dabei die Ultraschallausbreitung durch eventuell vorhandene Kleidungsstücke gedämpft.

Wird der Luftschallweg gewählt, ist anstelle der Laufzeitmessung von Ultraschallimpulsen auch eine Phasendifferenzmessung zwischen gesendetem und empfangenem Signal zur Abstandsermittlung vorteilhaft.

Eine spezielle Anwendung bezieht sich auf die Erfassung der Winkelstellung von Gelenken. Dabei spielt die richtige Wahl der Ultraschallwandlerpositionen eine besondere Rolle, da an Gelenken die Ultraschallausbreitung oft durch Knochengewebe gestört wird. Im Knochen ist sowohl die Ultraschallabsorption als auch die Schallgeschwindigkeit höher als im Weichteilgewebe.

Zur Messung am Knie wird vorzugsweise der Schallweg medial der Kniescheibe bei einem Ultraschallwandlerabstand von wenigen Zentimetern gewählt.

## Patentansprüche

1. Verfahren zur Messung der Körperhaltung und/oder -bewegung, wobei die Haltungs- und Bewegungsparameter mittels auf der Haut applizierter Sender-Empfänger-Systeme erfaßt werden, wobei die Sender-Empfänger-Systeme aus wenigstens einem Sender und wenigstens einem Empfänger bestehen, die voneinander beabstandet anbringbar sind, und wobei der Sender eine Welle sendet, die vom Empfänger empfangen wird, gekennzeichnet dadurch, daß
- zumindest ein Ultraschall- oder elektromagnetisches Sender-Empfänger-System (1) verwendet wird,
- daß eine Elektronikeinheit (2) eine abstandsabhängige Größe des empfangenen Signals ermittelt, so daß die Abstandsänderung zwischen Sender und Empfänger gemessen wird,
- und daß die Wellenlänge des Sender-Empfänger-Systems (1) so ausgewählt ist, daß eine durch die Dehnung oder Kontraktion der Haut ausgelöste Abstandsänderung zwischen Sender und Empfänger ermittelt werden kann, wenn das Sender-Empfänger-System auf solchen vorbestimmbaren Hautpunkten (4) angebracht wird, deren Abstand sich während der zu messenden Körperbewegung aufgrund der Hautelastizität ändert.

2. Verfahren nach Anspruch 1, dadurch gekennzeichnet, daß als Sender und Empfänger Ultraschallwandler eingesetzt werden und als abstandsabhängige Größe die Ultraschall-Laufzeit zwischen den Ultraschallwandlern verwendet wird.

3. Verfahren nach Anspruch 2, dadurch gekennzeichnet, daß zur Bestimmung der Haltungs- und Bewegungsparameter die Ultraschallwandler auf der Haut mit einer Ultraschalleinkopplung unter einem flachen Winkel zur Hautoberfläche anbringbar sind, und daß ein oberflächlich durch die Körperschichten durchlaufener Weg zwischen genannten Ultraschallwandlern als Meßstrecke verwendbar ist.

4. Verfahren nach Anspruch 1, dadurch gekennzeichnet, daß als Sender und Empfänger wenigstens ein elektromagnetischer Sender und wenigstens ein elektromagnetischer Empfänger eingesetzt werden, wobei als abstandsabhängige Größe die Intensität der elektromagnetischen Welle bzw. des elektromagnetischen Feldes verwendet wird.

5. Verfahren nach Anspruch 1, dadurch gekennzeichnet, daß als Sender und Empfänger wenigstens ein elektromagnetischer Sender und wenigstens ein elektromagnetischer Empfänger eingesetzt werden, wobei als abstandsabhängige Größe die Phasendifferenz zwischen gesendetem und empfangenem Signal verwendet wird.

6. Verfahren nach Anspruch 5, dadurch gekennzeichnet, daß die zum Einsatz gelangende Wellenlänge in der Größenordnung des Abstandes zwischen genanntem Sender und genanntem Empfänger festgelegt wird.

7. Vorrichtung zur Verbesserung der Körperhaltung und/oder - bewegung eines Patienten oder Probanden unter Verwendung des Verfahrens gemäß einem der Ansprüche 1-6 im Sinne eines Biofeedback, dadurch gekennzeichnet,
- daß die Vorrichtung zumindest ein Ultraschall- oder elektromagnetisches Sender-Empfänger-System (1) umfaßt, wobei die Sender-Empfänger-Systeme aus einem Sender und einem Empfänger bestehen, die voneinander beabstandet auf ersten und zweiten vorbestimmten Hautpunkten anbringbar sind, welche bei Körperbewegung eine Abstandsänderung zwischen Sender und Empfänger bewirken, und wobei der Sender eine Welle sendet, die vom Empfänger empfangen wird,
- daß eine Elektronikeinheit (2) zur Ermittlung einer abstandsabhängigen Größe des empfangenen Signals vorgesehen ist, so daß die Abstandsänderung zwischen Sender und Empfänger gemessen wird,
- daß die Vorrichtung ein mikroprozessorgesteuertes Gerät ist und eine Signalisierungsvorrichtung umfaßt, die angesteuert wird, wenn die abstandsabhängige Größe einen vom Arzt oder einer anderen Person vorgegebenen Wert überschreitet.

8. Vorrichtung nach Anspruch 7 unter Verwendung des Verfahrens gemäß einem der Ansprüche 1 bis 3, dadurch gekennzeichnet, daß für genannte Sender und Empfänger Ultraschallwandler eingesetzt werden und die Abstandsmessung durch eine Ultraschall-Laufzeit-Bestimmung auf einem durch die oberflächlichen Körperschichten verlaufenden Weg zwischen genannten Ultraschallwandlern erfolgt.

## Claims

1. Method for measuring the body posture and/or body movement in which the posture and movement parameters are detected by means of transmitter-receiver units applied to the skin, said transmitter-receiver units are constituted of at least one transmitter and one receiver being applicable at spaced relation to one another, said transmitter transmitting at least one wave for being detected by said receiver, characterized in that
- at least one ultrasonic or electromagnetic transmitter-receiver unit (1) is employed,
- an electronic unit (2) detects a space dependent value from the signal received thus measuring the space variation between transmitter and receiver,
- the wavelength of the transmitter-receiver unit (1) is so selected that it is feasible to measure a space variation between transmitter and receiver due to expansion or contraction of the skin, provided that the transmitter-receiver unit is attached to such preselected loci of the skin (4), the space between said loci varying during the body movement to be measured due to the skin elasticity.

2. Method as claimed in claim 1, wherein ultrasonic transducers are employed as transmitter and receiver and the ultrasonic propagation time between the ultrasonic transducers is evaluated as space dependent value.

3. Method as claimed in claim 2, wherein, in order to determine the posture and movements parameters, the ultrasonic transducers are attachable under a small angle relative to the skin surface for ultrasonic coupling, and wherein a path between said ultrasonic transducers is employed as measuring path, said path running through near-surface laminae.

4. Method as claimed in claim 1, wherein at least one electromagnetic transmitter and at least one electromagnetic receiver are employed as transmitter and receiver, the intensity of the electromagnetic wave and of the electromagnetic field, respectively, being exploited as space dependent value.

5. Method as claimed in claim 1, wherein at least one electromagnetic transmitter and at least one electromagnetic receiver are employed as transmitter and receiver, the phase shift between transmitted and received signal being exploited as space dependent value.

6. Method as claimed in claim 5, wherein the wavelength employed is set to the order of magnitude of the space between said transmitter and said receiver.

7. A device for improving the posture and/or the body movement of a patient or person under test under use of the method according to one of the preceding claims 1 to 6 in the meaning of a biofeedback, characterized in that
- the device comprises at least one ultrasonic or electromagnetic transmitter-receiver unit (1), said transmitter-receiver unit being constituted of a transmitter and a receiver attached in spaced relation on respective first and second preselected loci on the skin which, due to body movements, effect a space variation between transmitter and receiver, and in that said transmitter sends a wave for being received by the receiver,
- an electronic unit (2) is provided for detecting a space dependent value from the received signal, thus measuring the space variation between the transmitter and the receiver,
- the device is a microprocessor-controlled unit including a signal element being actuated when the space dependent value exceeds a value preset by the physician or any other person.

8. Device as claimed in claim 7 under use of the method according to the claims 1 to 3, wherein ultrasonic transducers are employed as transmitter and receiver and the space measurement is carried out by an ultrasonic run-time determination along a path through the near-surface laminae between said ultrasonic transducers.

## Revendications

1. Procédé destiné à la mesure de la tenue et/ou du mouvement du corps; les paramètres de tenue et de mouvement sont saisis au moyen de systèmes d'émetteur-récepteur placés sur la peau; les systèmes d'émetteur-récepteur sont constitués d'au moins un émetteur et d'au moins un récepteur, ceux-ci (émetteur et récepteur) pouvant être fixés à une certaine distance l'un de l'autre; l'émetteur émet une onde qui est reçue par le récepteur; le procédé est caractérisé par le fait que
- au moins un système d'émetteur-récepteur ultrasonore ou électromagnétique (1) est utilisé,
- qu'une unité électronique (2) saisit une grandeur du signe reçu, dépendan te de l'écart, ce qui permet de mesurer la modification de l'écart entre l'émet teur et le récepteur,
- et que la longueur d'ondes du système d'émetteur-récepteur (1) est choisie de manière à pouvoir saisir une modification de l'écart entre l'émetteur et le récepteur provoquée par l'extension ou la contraction de la peau, lorsque le système d'émetteur-récepteur est fixé à de tels points cutanés prédéterminables (4) dont l'écart (entre les points) se modifie, en raison de l'élasticité de la peau, pendant le mouvement corporel à mesurer.

2. Procédé selon la spécification 1, caractérisé par le fait que des transducteurs ultrasonores sont utilisés en tant qu'émetteur et récepteur, et caractérisé par fait que la durée de parcours des ondes ultrasonores est employée en tant que grandeur dépendante de l'écart.

3. Procédé selon la spécification 2, caractérisé par le fait que, pour la détermination des paramètres de tenue et de mouvement, les transducteurs ultrasonores peuvent être placés sur la peau à l'aide d'une alimentation en ultrasons sous un angle plan par rapport à la surface cutanée; le procédé est en outre caractérisé par le fait que le chemin parcouru à travers les couches superficielles du corps, entre les transducteurs ultrasonores mentionnés, peut être utilisé en tant que parcours de mesure.

4. Procédé selon la spécification 1, caractérisé par le fait qu'au moins un émetteur électromagnétique et au moins un récepteur électromagnétique sont employés en tant qu'émetteur et récepteur, l'intensité de l'onde électromagnétique ou bien du champ électromagnétique étant utilisée comme grandeur dépendante de l'écart.

5. Procédé selon la spécification 1, caractérisé par le fait qu'au moins un émetteur électromagnétique et au monts un récepteur électromagnétique sont employés en tant qu'émetteur et récepteur, la différence de phase entre les signes émis et reçu étant utilisée comme grandeur dépendante de l'écart.

6. Procédé selon la spécification 5, caractérisé par le fait que la longueur d'ondes employée est déterminée suivant l'ordre de grandeur de l'écart entre l'émetteur mentionné et le récepteur mentionné.

7. Dispositif servant à améliorer la tenue et/ou le mouvement du corps d'un patient ou d'un propositus, en appliquant le procédé conformément à une des spécifications 1 à 6, au sens d'une bio-rétroaction, caractérisé par le fait
- que le dispositif comprend au moins un système d'émetteur-récepteur (1) ultrasonore ou électromagnétique; les systèmes d'émetteur-récepteur sont constitués par un émetteur et un récepteur pouvant être fixés, à une certaine distance l'un de l'autre, sur des points cutanés premiers et deuxièmes, qui sont des points prédéterminés; lors d'un mouvement du corps, ces points provoquent une modification de l'écart entre l'émetteur et le récepteur, l'émetteur émettant une onde qui est reçue par le récepteur,
- qu'une unité électronique (2) est prévue pour établir la grandeur, dépendante de l'écart, du signe reçu, ce qui permet de mesurer la modification de l'écart entre l'émetteur et le récepteur,
- que le dispositif représente un appareil commandé par microprocesseur comprenant un dispositif de signalisation qui est amorcé lorsque la grandeur dépendante de l'écart dépasse une valeur déterminée par le médecin ou par une autre personne.

8. Dispositif selon la spécification 7, avec l'emploi du procédé conformément à une des spécifications 1 à 3, caractérisé par le fait que des transducteurs ultrasonores sont utilisés pour les émetteurs et les récepteurs mentionnés; la mesure de l'écart s'effectue grâce à la détermination de la durée de parcours des ondes ultrasonores sur la voie à travers les couches superficielles du corps, entre les transducteurs ultrasonores mentionnés.
